(19) Europäisches Patentamt
European Patent Office
Office européen des brevets

(11) Veröffentlichungsnummer: **0 254 150 A1**

(12) # EUROPÄISCHE PATENTANMELDUNG

(21) Anmeldenummer: 87109963.6

(22) Anmeldetag: 10.07.87

(51) Int.Cl.³: C 07 C 87/44
C 07 D 265/30, C 07 D 211/14
C 07 D 295/02, A 01 N 33/04
A 01 N 43/84

(30) Priorität: 22.07.86 DE 3624648

(43) Veröffentlichungstag der Anmeldung:
27.01.88 Patentblatt 88/4

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Weissmüller, Joachim, Dr.
Carl-Langhans-Strasse 53
D-4019 Monheim(DE)

(72) Erfinder: Reinecke, Paul, Dr.
Steinstrasse 8
D-5090 Leverkusen 3(DE)

(54) **Decahydronapht-2-yl-alkylamine.**

(57) Decahydronaphth-2-yl-alkylamine der allgemeinen Formel

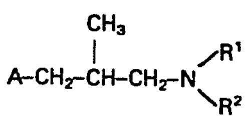

in welcher

A für gegebenenfalls substituiertes Decahydronaphth-2-yl steht und

$R^1$ und $R^2$ gleich oder verschieden sind und für Alkyl oder Alkenyl stehen, oder

$R^1$ und $R^2$ gemeinsam mit dem Stickstoffatom, an das sie gebunden sind, für einen gegebenenfalls substituierten, gesättigten Heterocyclus stehen, der weitere Heteroatome enthalten kann,

und deren pflanzenverträgliche Säureadditions-Salze, ein Verfahren zu deren Herstellung, sowie ihre Verwendung als Fungizide.

EP 0 254 150 A1

Croydon Printing Company Ltd.

0254150

BAYER AKTIENGESELLSCHAFT 5090 Leverkusen, Bayerwerk
Konzernverwaltung RP
Patentabteilung

Bas/Sdt/ABc
Ia

## Decahydronaphth-2-yl-alkylamine

Die Erfindung betrifft neue Decahydronaphth-2-yl-alkylamine, ein Verfahren zu ihrer Herstellung und ihre Verwendung als Fungizide.

Es ist bereits bekannt, daß bestimmte β-Naphthylalkylamine, wie beispielsweise das cis-1-(2,6-Dimethyl-morpholin-4-yl)-2-methyl-3-(6-methyl-naphth-2-yl)-propan, fungizide Eigenschaften besitzen (vgl. DE-OS 34 13 897). Die Wirkung dieser Verbindungen ist jedoch unter bestimmten Bedingungen, insbesondere bei niedrigen Aufwandmengen und Konzentrationen, nicht immer voll befriedigend.

Es wurden neue Decahydronaphth-2-yl-alkylamine der allgemeinen Formel (I)

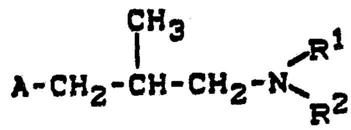 (I)

in welcher

A für gegebenenfalls substituiertes Decahydronaphth-2-yl steht und

$R^1$ und $R^2$ gleich oder verschieden sind und für Alkyl oder Alkenyl stehen, oder

$R^1$ und $R^2$ gemeinsam mit dem Stickstoffatom, an das sie gebunden sind, für einen gegebenenfalls substituierten, gesättigten Heterocyclus stehen, der weitere Heteroatome enthalten kann,

sowie deren pflanzenverträgliche Säureadditions-Salze gefunden.

Weiterhin wurde gefunden, daß man die neuen Decahydronaphth-2-yl-alkylamine der allgemeinen Formel (I)

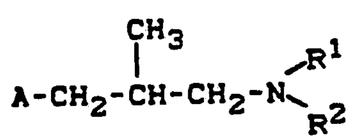 (I)

in welcher

A für gegebenenfalls substituiertes Decahydronaphth-2-yl steht und

$R^1$ und $R^2$ gleich oder verschieden sind und für Alkyl oder Alkenyl stehen, oder

$R^1$ und $R^2$ gemeinsam mit dem Stickstoffatom, an das sie gebunden sind, für einen gegebenenfalls substituierten, gesättigten Heterocyclus stehen, der weitere Heteroatome enthalten kann,

sowie deren pflanzenverträgliche Säureadditions-Salze erhält, wenn man

β-Naphthylalkylamin-Verbindungen der allgemeinen Formel (II)

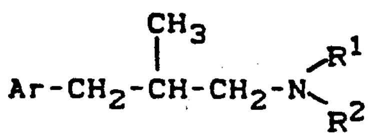
(II)

in welcher

Ar für gegebenenfalls substituiertes β-Naphthyl steht und

$R^1$ und $R^2$ die oben angegebene Bedeutung haben,

mit Wasserstoff, gegebenenfalls in Gegenwart eines Katalysators und gegebenenfalls in Gegenwart eines Verdünnungsmittels, gegebenenfalls unter Druck hydriert, und gegebenenfalls anschließend eine Säure addiert.

Die neuen Decahydronaphth-2-yl-alkylamine der Formel (I) enthalten chirale Zentren und werden im allgemeinen in Form von Mischungen (Racemate oder Diastereomerengemische)

erhalten. Die Diastereomeren lassen sich gegebenenfalls in allgemein bekannter Art und Weise in reiner Form isolieren, wie beispielsweise durch Säulenchromatographie oder aufgrund von Löslichkeitsunterschieden. Aus solchen Diastereomeren kann man mit bekannten Methoden einheitliche Enantiomere erhalten. Diese werden wie ihre Mischungen von der vorliegenden Erfindung umfaßt.

Schließlich wurde gefunden, daß die neuen Decahydronaphth-2-yl-alkylamine der Formel (I) gute fungizide Eigenschaften besitzen. Dabei zeigen die erfindungsgemäßen Verbindungen überraschenderweise eine bessere Wirksamkeit, als die aus dem Stand der Technik bekannten β-Naphthyl-alkylamine, wie beispielsweise das cis-1-(2,6-Dimethylmorpholin-4-yl)-2-methyl-3-(6-methyl-naphth-2-yl)-propan, welches chemisch und wirkungsmäßig naheliegende Verbindungen sind.

Die erfindungsgemäßen Decahydronaphth-2-yl-alkylamine sind durch die Formel (I) allgemein definiert. Bevorzugt sind Verbindungen der Formel (I), bei welchen

A für gegebenenfalls einfach bis dreifach, gleich oder verschieden substituiertes Decahydronaphth-2-yl steht, wobei als Substituenten genannt seien: Hydroxy, jeweils geradkettiges oder verzweigtes Alkyl und Alkoxy mit jeweils 1 bis 6 Kohlenstoffatomen;

$R^1$ und $R^2$ gleich oder verschieden sind und für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoffatom, an welches sie gebunden sind, für einen gegebenenfalls ein- bis dreifach, gleich oder verschieden substituierten, gesättigten fünf- bis siebengliedrigen Heterocyclus stehen, der 1 oder 2 weitere Heteroatome, insbesondere Stickstoff oder Sauerstoff enthalten kann, wobei als Substituenten in Frage kommen: jeweils geradkettiges oder verzweigtes Alkyl oder Hydroxyalkyl mit jeweils 1 bis 4 Kohlenstoffatomen.

Besonders bevorzugt sind Decahydronaphth-2-yl-alkylamine der Formel (I), bei welchen

A für gegebenenfalls einfach oder zweifach, gleich oder verschieden substituiertes Decahydronaphth-2-yl steht, wobei als Substituenten genannt seien: Hydroxy, Methyl, Ethyl, n- oder i-Propyl; n-, i-, s- oder t-Butyl, Methoxy, Ethoxy, n- oder i-Propoxy,

$R^1$ und $R^2$ gleich oder verschieden sind und für Methyl, Ethyl, n- oder i-Propyl und n-, i- oder s-Butyl stehen; oder für Allyl, Butenyl, Dimethylallyl, n- oder i-Pentenyl stehen, oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

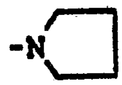 , 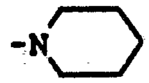 , 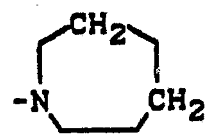 oder 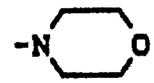

stehen, wobei als Substituenten infrage kommen: Methyl, Ethyl oder Hydroxymethyl.

Ganz besonders bevorzugt sind Decahydronaphth-2-ylalkylamine der Formel (I), bei welchen

A für gegebenenfalls einfach oder zweifach, gleich oder verschieden substituiertes Decahydronaphth-2-yl steht, wobei als Substituenten infrage kommen: Methyl, und

$R^1$ und $R^2$ gemeinsam mit dem Stickstoffatom, an welches sie gebunden sind, für einen gegebenenfalls ein- bis dreifach durch Methyl substituierten Heterocyclus der Formel

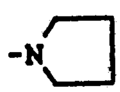 , 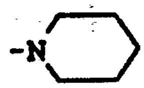 , 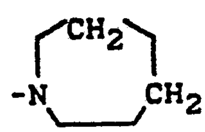 oder 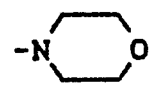

stehen.

Im einzelnen seien außer den bei den Herstellungsbeispielen genannten Verbindungen die folgenden Decahydronaphth-2-yl-alkylamine der Formel (I) genannt:

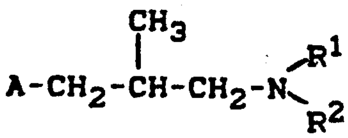 (I)

| A | $R^1$ | $R^2$ bzw. 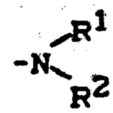 |
|---|---|---|
| 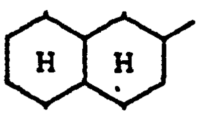 | $CH_3$ | $-CH_2-CH=CH_2$ |
| 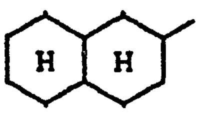 | - | - 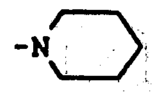 |
| 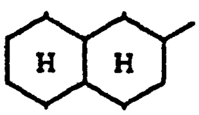 | - | - 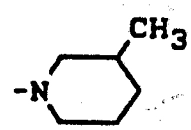 |
| 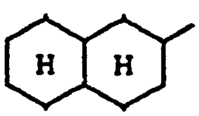 | - | - 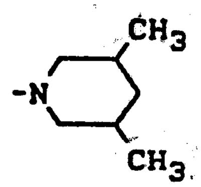 |
| 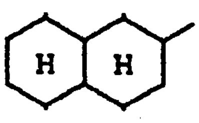 | $CH_3$ | 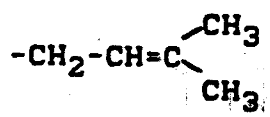 |
| 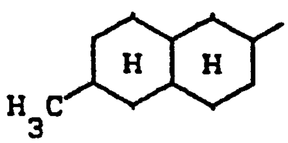 | - | - 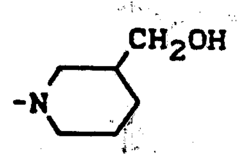 |
| 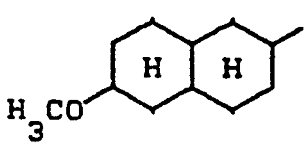 | - | - 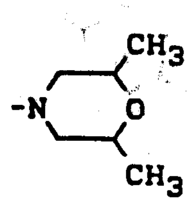 |

| A | $R^1$ | $R^2$ | bzw. $-N<^{R^1}_{R^2}$ |
|---|---|---|---|
| 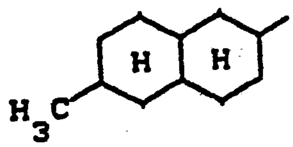 | - | - | 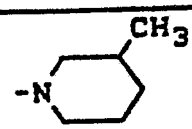 |
| 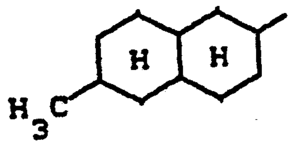 | - | - | 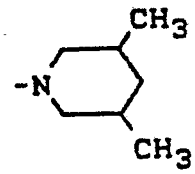 |
| 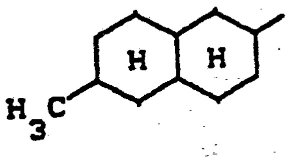 | $CH_3$ | - | 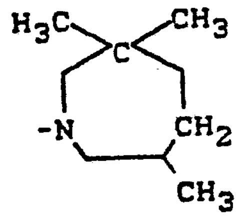 |
| 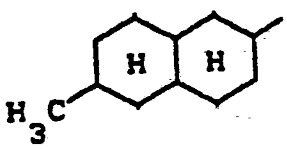 | $CH_3$ | $-(CH_2)_4-CH_3$ | |
| 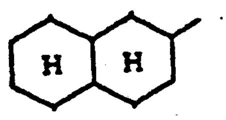 | $CH_3$ | 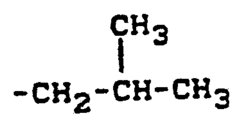 | |
| 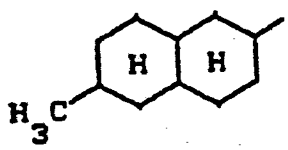 | - | - | 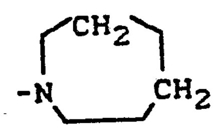 |
| 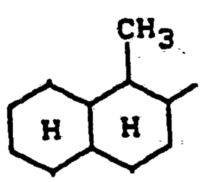 | - | - | 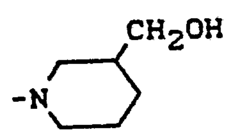 |

Verwendet man beispielsweise cis-1-(2,6-Dimethylmorpholin-4-yl)-2-methyl-3-β-(6-methylnaphthyl)-propan als Ausgangsstoff, so läßt sich der Reaktionsablauf des erfindungsgemäßen Verfahrens durch das folgende Formelschema darstellen:

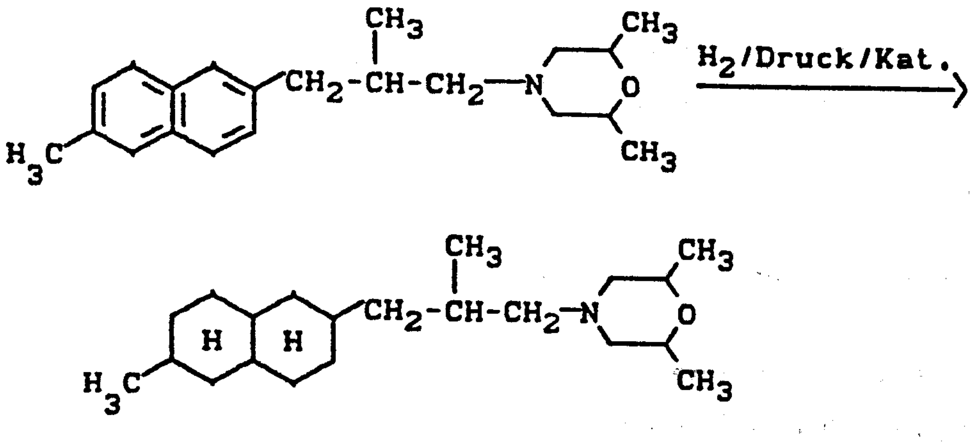


Die zur Durchführung des erfindungsgemäßen Verfahrens als Ausgangsstoffe benötigten β-Naphthyl-alkylamin-Verbindungen sind durch die Formel (II) allgemein definiert. In dieser Formel stehen $R^1$ und $R^2$ vorzugsweise für diejenigen Bedeutungen, die bereits im Zusammenhang mit der Beschreibung der erfindungsgemäßen Stoffe der Formel (I) vorzugsweise für diese Substituenten genannt wurden. Ar steht vorzugsweise für gegebenenfalls einfach bis dreifach, gleich oder verschieden substituiertes β-Naphthyl, wobei als Substituenten vorzugsweise genannt seien: Hydroxy; geradkettiges oder verzweigtes Alkyl und Alkoxy mit jeweils 1 bis 6 Kohlenstoffatomen.

Die β-Naphthylalkylamin-Verbindungen der Formel (II) sind bekannt (vgl. DE-OS 34 13 897) bzw. lassen sie sich nach den dort beschriebenen Verfahren erhalten, indem man z.B. β-Naphthyl-Derivate der allgemeinen Formel (III)

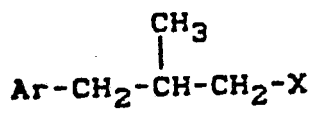

(III)

in welcher

Ar die oben angegebene Bedeutung hat und

X für eine elektronenanziehende Austrittsgruppe, wie beispielsweise Methansulfonyloxy, p-Toluolsulfonyloxy oder Halogen steht,

mit bekannten Aminen der allgemeinen Formel (IV)

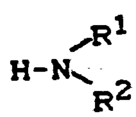

(IV)

in welcher

$R^1$ und $R^2$ die oben angegebene Bedeutung haben,

gegebenenfalls in Gegenwart eines Verdünnungsmittels, wie beispielsweise Ether oder Amide, und gegebenenfalls in Gegenwart eines Säurebindemittels, wie beispielsweise tertiäre Amine, bei Temperaturen zwischen 30° C und 180° C umsetzt.

Die β-Naphthyl-Derivate der Formel (III) sind bekannt (vgl. DE-OS 34 13 897) bzw. lassen sich nach den dort beschriebenen Verfahren erhalten, wenn man 2-Methyl-3-β-naphthyl-acryl-ester der allgemeinen Formel (V)

$$\begin{array}{l} \qquad\qquad\quad CH_3 \\ \qquad\qquad\quad | \\ Ar-CH=C-CO-O-R^3 \end{array} \qquad (V)$$

in welcher

Ar die oben angegebene Bedeutung hat und

$R^3$ für Alkyl, insbesondere für Methyl oder Ethyl steht,

zunächst in einer 1. Stufe mit einem Reduktionsmittel, wie beispielsweise Lithiumaluminiumhydrid, gegebenenfalls in Gegenwart eines Verdünnungsmittels, wie beispielsweise Diethylether, bei Temperaturen zwischen -20° C und +60° C reduziert, und die so erhaltenen Alkohole der allgemeinen Formel (VI),

$$\begin{array}{l} \qquad\qquad\quad\;\; CH_3 \\ \qquad\qquad\quad\;\; | \\ Ar-CH_2-CH-CH_2-OH \end{array} \qquad (VI)$$

in welcher

Ar die oben angegebene Bedeutung hat,

nach allgemein üblichen Verfahren derivatisiert, so zum Beispiel entweder mit Sulfonsäurehalogeniden der allgemeinen Formel (VII)

$R^4$-$SO_2$-Hal (VII)

in welcher

$R^4$ für jeweils gegebenenfalls substituiertes Alkyl oder Aryl, insbesondere für Methyl, Trifluormethyl oder p-Tolyl, steht, und

Hal für Halogen, insbesondere Chlor oder Brom, steht,

gegebenenfalls in Gegenwart eines Verdünnungsmittels, wie beispielsweise Dichlormethan, und gegebenenfalls in Gegenwart eines Säurebindemittels, wie beispielsweise Triethylamin, bei Temperaturen zwischen -20°C und +120°C sulfoniert oder in Gegenwart eines Halogenierungsmittels, wie Thionylchlorid, Phosphorpentachlorid, Phosphortribromid, Bromwasserstoffsäure oder Iodwasserstoffsäure, gegebenenfalls in Gegenwart eines Verdünnungsmittels, wie beispielsweise Tetrachlormethan, und gegebenenfalls in Gegenwart eines Katalysators, wie beispielsweise Pyridin, bei Temperaturen zwischen +20°C und +180°C halogeniert.

Die 2-Methyl-3-β-naphthylacrylester der Formel (V) sind bekannt (vgl. z.B. Indian J. Chem. Section B, 22B (4), 352-354 [1983], J.Org. Chemistry 33, 4351-4362 [1968] oder DE-OS 34 13 897) oder lassen sich nach prinzipiell bekannten Verfahren in einfacher analoger Weise herstellen (vgl. z.B. J. Chem. Soc. 1961, 3160). Die Sulfonsäurehalogenide der Formel (VII) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören vorzugsweise aliphatische Kohlenwasserstoffe, wie Petrolether, Hexan oder Cyclohexan; Ether, wie Diethylether, Dioxan, Tetrahydrofuran oder Ethylenglykoldimethyl- oder -diethylether; Ester, wie Essigsäureethylester; Sulfoxide, wie Dimethylsulfoxid; sowie Alkohole, wie Methanol oder Ethanol.

Als Katalysatoren zur Durchführung des erfindungsgemäßen Verfahrens kommen übliche Hydrierkatalysatoren in Frage. Vorzugsweise verwendet man Edelmetall-, Edelmetalloxid- oder Edelmetallhydroxid-Katalysatoren bzw. sogenannte Raney-Katalysatoren, wie insbesondere Platin, Platinoxid, Nickel und Ruthenium.

Die Reaktionstemperaturen können bei der Durchführung des erfindungsgemäßen Verfahrens in einem größeren Bereich variiert werden. Im allgemeinen arbeitet man bei Temperaturen zwischen 20° C und 250° C, vorzugsweise bei Temperaturen zwischen 20° C und 200° C.

Das erfindungsgemäße Verfahren kann bei Normaldruck oder auch bei erhöhtem Druck durchgeführt werden. Im allgemeinen arbeitet man zwischen 1 atm und 300 atm, vorzugsweise bei 1 atm bis 200 atm.

Es ist jedoch auch möglich, die neuen Decahydronaphth-2-ylalkylamine der Formel (I) nach einem anderen Verfahren

herzustellen, indem man z.B. die Alkohole der Formel (VI) analog dem oben angegebenen erfindungsgemäßen Verfahren hydriert; die entsprechenden Decahydronaphth-2-yl-Derivate

$$(A-CH_2-\overset{CH_3}{\overset{|}{C}}H-CH_2OH)$$

nach allgemein üblichen Verfahren derivatisiert und die so erhaltenen Verbindungen

$$(A-CH_2-\overset{CH_3}{\overset{|}{C}}H-CH_2X)$$

mit den Aminen der Formel (IV) nach dem oben angegebenen Verfahren zu den neuen Verbindungen der Formel (I) umsetzt.

Die erfindungsgemäßen Verbindungen der Formel (I) können gegebenenfalls anschließend in Säureadditionssalze übergeführt werden.

Zur Herstellung von physiologisch verträglichen Säureadditionssalzen der Verbindungen der Formel (I) kommen vorzugsweise folgende Säuren in Frage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, Sulfonsäuren, wie z.B. p-Toluolsulfonsäure und 1,5-Naphthalindisulfonsäure.

Bevorzugte Säureadditionsverbindungen sind dabei die Additionsverbindungen mit Chlorwasserstoff, p-Toluolsulfonsäure, Methansulfonsäure, 1,6-Dinaphthalinsulfonsäure, Ameisensäure und Essigsäure.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerte organischen Lösungsmittel gereinigt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So werden z.B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;

Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe können mit besonders gutem Erfolg zur Bekämpfung von Getreidekrankheiten, wie beispielsweise Erysiphe graminis, Septoria nodorum und Drechslera teres, ferner von echtem Mehltau und Phytophthora sowie von Pyricularia oryzae an Reis eingesetzt werden. Außerdem zeigen die erfindungsgemäßen Stoffe eine gute Wirkung im Myzelwachstumstest.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid, N-Methylpyrrolidon und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie

Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit andren bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen

wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

**Herstellungsbeispiele**

**Beispiel 1**

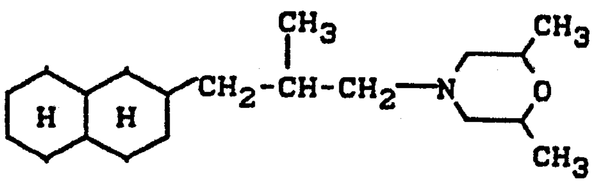

10 g (0,033 Mol) 1-[cis-2,6-Dimethylmorpholin-4-yl]-2-methyl-3-β-naphthyl-propan werden in 150 ml Tetrahydrofuran bei 120° C in Gegenwart von 3 g Ruthenium-Kohlenstoff (5 %) und bei einem Wasserstoffdruck von 150 bar 3 Stunden hydriert. Vom Katalysator wird abfiltriert und das Lösungsmittel im Vakuum verdampft.
Man erhält 7,7 g (74,6 % der Theorie) an 1-[cis-2,6-Dimethylmorpholin-4-yl]-2-methyl-3-[decahydronaphth-2-yl]-propan vom Brechungsindex $n_D^{20}$ = 1,4869.

Herstellung der Ausgangsverbindung

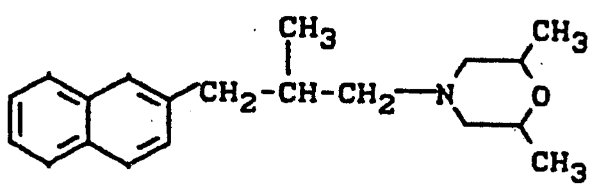

10,8 g (0,04 Mol) 1-Methansulfonyloxy-2-methyl-3-β-naphthyl-propan und 9 g (0,078 Mol) cis-2,6-Dimethylmorpholin werden bei einer Badtemperatur von 140° C 15 Stunden gerührt. Die erhaltene Reaktionsmischung wird mit Wasser versetzt

und mehrfach mit Ether extrahiert. Die vereinigten organischen Phasen werden über Natriumsulfat getrocknet und im Vakuum vom Lösungsmittel befreit; der ölige Rückstand wird säulenchromatographisch (Kieselgel 60, Petrolether/Essigester = 2 : 1) gereinigt.
Man erhält 6,2 g (52 % der Theorie) an cis-1-(2,6-Dimethylmorpholin-4-yl)-2-methyl-3-β-naphthyl-propan vom Brechungsindex $n_D^{20}$ = 1,5527.

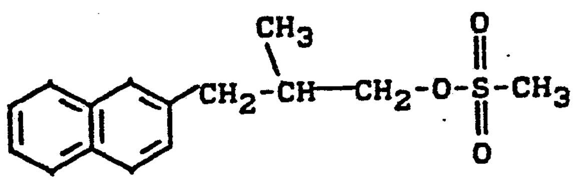

Zu 14 g (0,074 Mol) 2-Methyl-3-β-naphthylpropanol (roh) in 80 ml absolutem Pyridin gibt man bei 0°C tropfenweise unter Rühren 11 g (0,1 Mol) Methansulfonsäurechlorid, rührt nach beendeter Zugabe weitere 16 Stunden bei Raumtemperatur, entfernt überschüssiges Pyridin durch Destillation im Vakuum, nimmt den Rückstand in Wasser auf, extrahiert mehrfach mit Dichlormethan, trocknet über Natriumsulfat und entfernt das Lösungsmittel im Vakuum. Man erhält 13,6 g (66 % der Theorie) an 1-Methansulfonyloxy-2-methyl-3-β-naphthyl-propan als Öl.
(IR: $\nu$ = 1345, 1180 $cm^{-1}$).

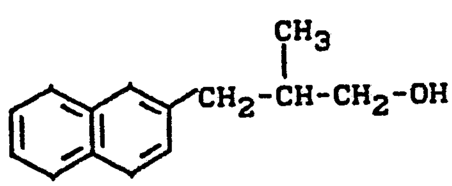

12 g (0,05 Mol) 2-Methyl-3-β-naphthylacrylsäureethylester tropft man unter Eiskühlung in einer trockenen Stickstoffatmosphäre zu einer Suspension von 1,9 g (0,05 Mol) Lithiumaluminiumhydrid in 150 ml absolutem Ether. Nach beendeter Zugabe erwärmt man 8 Stunden lang auf Rückflußtemperatur und tropft dann nach Erkalten der Reaktionsmischung langsam unter Kühlung 15 ml 5-%ige Schwefelsäure zu, saugt den ausgefallenen Feststoff ab, trocknet das Filtrat über Natriumsulfat, entfernt das Lösungsmittel im Vakuum und kristallisiert den Rückstand aus Ether/Petrolether um. Man erhält 7,1 g 2-Methyl-3-β-naphthyl-propanol vom Schmelzpunkt 71-74° C, das laut Gaschromatogram mit 2-Methyl-1-β-naphthyl-propen-3-ol verunreinigt ist und ohne weitere Reinigung in die nächste Reaktionsstufe eingesetzt wird.

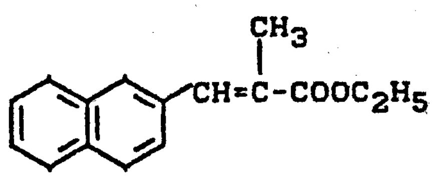

Zu einer Suspension von 5,5 g (0,2 Mol) 80 %igem Natriumhydrid in 300 ml absolutem Xylol gibt man bei 70° C 40 g (0,2 Mol) Ethyl-α-ethoxalylpropionat. Nach beendeter Wasserstoffentwicklung tropft man 31,2 g (0,2 Mol) β-Naphthaldehyd in Xylol gelöst zu und erwärmt nach beendeter Zugabe für 90 Minuten zum Sieden.

Die erkaltete Reaktionsmischung wird mit 150 ml Wasser versetzt, die organische Phase abgetrennt, mit 7-%iger

Natriumcarbonatlösung gewaschen, über Natriumsulfat getrocknet und eingeengt. Der Rückstand wird im Vakuum destilliert. Man erhält 21,7 g (45,2 % der Theorie) an 2-Methyl-3-β-naphthyl-acrylsäureethylester vom Siedepunkt 110° C/0,13 mbar.

In analoger Weise und gemäß den allgemeinen Verfahrensangaben werden die folgenden Verbindungen der allgemeinen Formel (I)

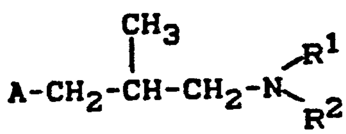 (I)

erhalten:

| Bsp. Nr. | A | 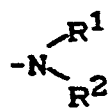 | Brechungsindex ($n_D^{20}$) |
|---|---|---|---|
| 2 | 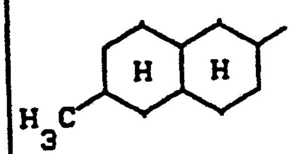 | 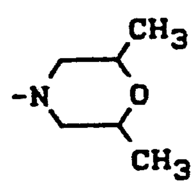 | 1,4954 |
| 3 | 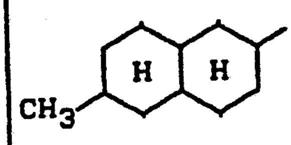 | 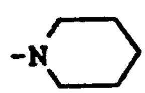 | 1,4941 |
| 4 | 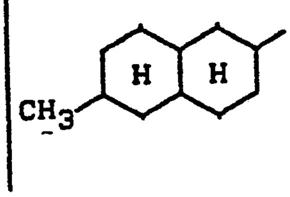 | 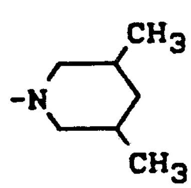 | 1,4881 |

Verwendungsbeispiel

In dem folgendem Verwendungsbeispiel wird die nachstehend angegebene Verbindung als Vergleichssubstanz eingesetzt:

(A)

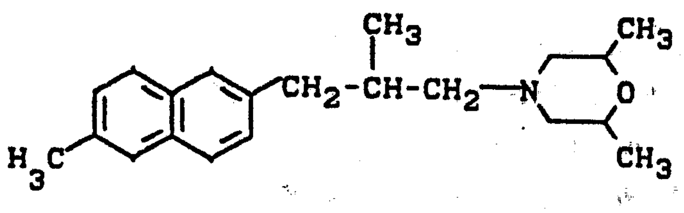

cis-1-(2,6-Dimethyl-morpholin-4-yl)-2-methyl-3-(6-methyl-naphth-2-yl)-propan.

**Beispiel A**

Erysiphe-Test (Gerste) / protektiv /

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Die Verbindungen gemäß der Erfindung zeigen bei diesem Test eine deutliche Überlegenheit in der Wirksamkeit gegenüber dem Stand der Technik.

**Patentansprüche**

1. Decahydronaphth-2-yl-alkylamine der allgemeinen Formel (I)

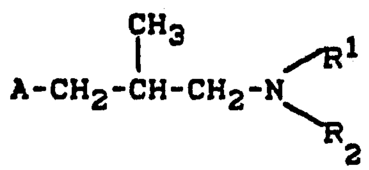

(I)

in welcher

A für gegebenenfalls substituiertes Decahydronaphth-2-yl steht und

$R^1$ und $R^2$ gleich oder verschieden sind und für Alkyl oder Alkenyl stehen, oder

$R^1$ und $R^2$ gemeinsam mit dem Stickstoffatom, an das sie gebunden sind, für einen gegebenenfalls substituierten, gesättigten Heterocyclus stehen, der weitere Heteroatome enthalten kann,

sowie deren pflanzenverträgliche Säureadditions-Salze.

2. Decahydronaphth-2-yl-alkylamine der allgemeinen Formel (I) gemäß Anspruch 1,

worin

A für gegebenenfalls einfach bis dreifach, gleich oder verschieden durch Hydroxy, jeweils geradkettiges oder verzweigtes Alkyl oder Alkoxy mit jeweils 1 bis 6 Kohlenstoffatomen substituiertes Decahydronaphth-2-yl steht,

$R^1$ und $R^2$ gleich oder verschieden sind und für jeweils geradkettiges oder verzweigtes Alkyl oder Alkenyl mit jeweils bis zu 6 Kohlenstoffatomen stehen oder

$R^1$ und $R^2$ gemeinsam mit dem Stickstoffatom, an welches sie gebunden sind, für einen gegebenenfalls ein- bis dreifach, gleich oder verschieden durch jeweils geradkettiges oder verzweigtes Alkyl oder Hydroxyalkyl mit jeweils 1 bis 4 Kohlenstoffatomen substituierten, gesättigten fünf- bis siebengliedrigen Heterocyclus stehen, der 1 oder 2 weitere Heteroatome in Form von Stickstoff oder Sauerstoff enthalten kann.

3. Decahydronaphth-2-yl-alkylamine der allgemeinen Formel (I) gemäß Anspruch 1, worin

A für gegebenenfalls einfach oder zweifach, gleich oder verschieden durch Hydroxy, Methyl, Ethyl, n- oder i-Propyl; n-, i-, s- oder t-Butyl, Methoxy, Ethoxy, n- oder i-Propoxy substituiertes Decahydronaphth-2-yl steht,

$R^1$ und $R^2$ gleich oder verschieden sind und für Methyl, Ethyl, n- oder i-Propyl und n-, i- oder s-Butyl stehen; oder für Allyl, Butenyl, Dimethylallyl, n- oder i-Pentenyl stehen, oder

$R^1$ und $R^2$ gemeinsam mit dem Stickstoffatom, an welches sie gebunden sind, für einen gegebenenfalls ein- bis dreifach, gleich oder verschieden durch Methyl, Ethyl oder Hydroxymethyl substituierten Heterocyclus der Formel

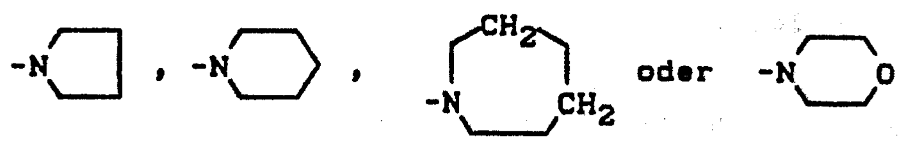

stehen.

4. Decahydronaphth-2-yl-alkylamine der allgemeinen Formel (I) gemäß Anspruch 1, worin

A für gegebenenfalls einfach oder zweifach durch Methyl substituiertes Decahydronaphth-2-yl steht, und

$R^1$ und $R^2$ gemeinsam mit dem Stickstoffatom, an welches sie gebunden sind, für einen gegebenenfalls ein- bis dreifach durch Methyl substituierten Heterocyclus der Formel

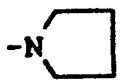 , 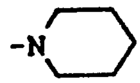 , 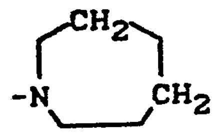 oder 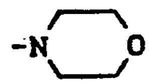

stehen.

5. Verfahren zur Herstellung von Decahydronaphth-2-yl-alkylaminen der allgemeinen Formel (I)

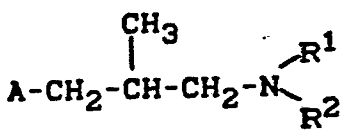 (I)

in welcher

A für gegebenenfalls substituiertes Decahydro-naphth-2-yl steht und

$R^1$ und $R^2$ gleich oder verschieden sind und für Alkyl oder Alkenyl stehen, oder

$R^1$ und $R^2$ gemeinsam mit dem Stickstoffatom, an das sie gebunden sind, für einen gegebenenfalls substituierten, gesättigten Heterocyclus stehen, der weiter Heteroatome enthalten kann,

sowie deren pflanzenverträgliche Säureadditions-Salzen, dadurch gekennzeichnet, daß man β-Naphthyl-alkylamin-Verbindungen der allgemeinen Formel (II)

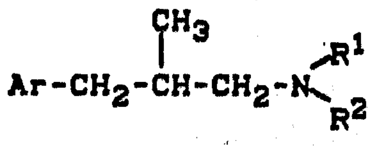 (II)

in welcher

Ar für gegebenenfalls substituiertes β-Naphthyl steht und

$R^1$ und $R^2$ die oben angegebene Bedeutung haben,

mit Wasserstoff, gegebenenfalls in Gegenwart eines Katalysators und gegebenenfalls in Gegenwart eines Verdünnungsmittels, gegebenenfalls unter Druck hydriert, und gegebenenfalls anschließend eine Säure addiert.

6. Fungizide Mittel, gekennzeichnet durch einen Gehalt an mindestens einer Verbindung der allgemeinen Formel (I) gemäß Anspruch 1.

7. Verfahren zur Behandlung von pilzlichen Schädlingen, dadurch gekennzeichnet, daß man mindestens eine Verbindung der allgemeinen Formel (I) gemäß Anspruch 1 auf pilzliche Schädlinge und/oder ihren Lebensraum einwirken läßt.

8. Verwendung von Verbindungen der allgemeinen Formel (I) gemäß Anspruch 1 zur Bekämpfung von pilzlichen Schädlingen.

9. Verfahren zur Herstellung von fungiziden Mitteln, dadurch gekennzeichnet, daß man Verbindungen der allgemeinen Formel (I) gemäß Anspruch 1 mit Streckmitteln und/oder oberflächenaktiven Mitteln vermischt.

Europäisches Patentamt

# EUROPÄISCHER RECHERCHENBERICHT

0254150

Nummer der Anmeldung

EP 87 10 9963

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.4) |
|---|---|---|---|
| Y | EP-A-0 019 769 (BASF)<br>* Ansprüche *<br>--- | 1,5-9 | C 07 C 87/44<br>C 07 D 265/30<br>C 07 D 211/14<br>C 07 D 295/02<br>A 01 N 33/04<br>A 01 N 43/84 |
| Y | EP-A-0 032 673 (BASF)<br>* Ansprüche *<br>--- | 1,5-9 | |
| D,Y | EP-A-0 161 455 (BAYER)<br>* Ansprüche *<br>----- | 1,5-9 | |
| | | | **RECHERCHIERTE SACHGEBIETE (Int. Cl.4)** |
| | | | C 07 C 87/00<br>C 07 D 211/00<br>C 07 D 265/00<br>C 07 D 295/00<br>A 01 N 33/00<br>A 01 N 43/00 |

Der vorliegende Recherchenbericht wurde für alle Patentansprüche erstellt

| Recherchenort | Abschlußdatum der Recherche | Prüfer |
|---|---|---|
| DEN HAAG | 02-11-1987 | MOREAU J.M. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)